**Europäisches Patentamt**

**European Patent Office**

**Office européen des brevets**

(19)

(11) Numéro de publication : **0 349 619 B1**

# (12) FASCICULE DE BREVET EUROPEEN

(45) Date de publication du fascicule du brevet :
**08.07.92 Bulletin 92/28**

(51) Int. Cl.$^5$ : **A61K 7/48,** A61K 7/06,
**C11D 3/384, C11D 9/40**

(21) Numéro de dépôt : **88910405.5**

(22) Date de dépôt : **08.12.88**

(86) Numéro de dépôt international :
**PCT/CH88/00223**

(87) Numéro de publication internationale :
**WO 89/05136 15.06.89 Gazette 89/13**

(54) **PRODUITS COSMETIQUES CONTENANT DES COMPOSANTS DU LAIT.**

(30) Priorité : **11.12.87 CH 4839/87**

(43) Date de publication de la demande :
**10.01.90 Bulletin 90/02**

(45) Mention de la délivrance du brevet :
**08.07.92 Bulletin 92/28**

(84) Etats contractants désignés :
**AT BE CH DE FR GB IT LI LU NL SE**

(56) Documents cités :
**EP-A- 0 046 326
CH-A- 85 649
FR-A- 1 039 726
GB-A- 2 050 160
US-A- 3 644 615
US-A- 4 223 018
Chemical Abstracts, volume 95, 1981, (Columbus, Ohio, US), M.P. Gupta et al.: "Effect of ions on the stability of caseinmicelles", see page 517, abstract 185704k, & Indian J. Dairy Sci. 1980, 33(3), 366-73**

(73) Titulaire : **RHONE-ELECTRA S.A.
12 bis, avenue de Rosemont
CH-1208 Genève (CH)**

(72) Inventeur : **GIDDEY, Claude
59, route de Chêne
CH-1208 Genève (CH)**
Inventeur : **BUNTER, Guy
28, avenue Vibert
CH-1227 Carouge (CH)**
Inventeur : **TZANOS, Dimitri
80, route de S.-Julien
CH-1212 Grand-Lancy (CH)**

(74) Mandataire : **Dousse, Blasco
7, route de Drize
CH-1227 Carouge/Genève (CH)**

EP 0 349 619 B1

## Description

La présente invention a pour objet des produits cosmétiques et/ou de savonnerie contenant des produits d'origine lactée, c'est-à-dire des ingrédients dérivés du lait entier ou écrémé ou extraits de celui-ci. L'invention concerne également la préparation de tels produits.

L'effet favorable sur les compositions cosmétiques, telles que lotions pour la peau, lotions capillaires, shampoings, crèmes adoucissantes, savons, de lait ou produits dérivés du lait est bien connu et de nombreuses compositions contenant de tels ingrédients ont été décrites dans l'état de la technique.

Ainsi, le document US-A-3,959,491 décrit des crèmes et lotions cosmétiques contenant du lait stérilisé et de la crème de lait. Pour améliorer la conservation de tels produits au stockage, ce document recommande d'incorporer aux compositions cosmétiques des acides gras saturés, des esters triglycéridiques d'acides gras saturés, des diols aliphatiques stabilisateurs, des agents séquestrants et des agents biocides, notamment des dénaturants d'enzymes et des bactéricides. Bien entendu, outre les agents de conservation ci-dessus, les cosmétiques décrits dans ce document contiennent les composants de cosmétiques habituels, y compris des parfums, des opacifiants et autres.

Le document DD-A-112.351 (Allemagne orientale) décrit des produits cosmétiques contenant un complexe actif dérivé de composants du lait ou du petit-lait, notamment de protéines non-dénaturées, vitamines et sels constituants du lait. Suivant un procédé de préparation d'un tel complexe actif décrit dans ce document, on soumet une masse de petit-lait à une concentration sous pression réduite suivie d'une centrifugation et, finalement, d'un séchage par pulvérisation. Pour la fabrication de cosmétiques, on ajoute à ceux-ci des proportions de l'ordre de 10 - 20% du complexe actif tel que préparé comme décrit.

Le document US-A-4,460,571 décrit un cosmétique à base de lait entier ou en poudre se prêtant plus particulièrement aux usages topodermiques et capillaires. Il s'agit là de compositions fluides, antiseptiques et parfumantes contenant du lait dégraissé ou non, ou du lait en poudre dissous et, outre l'eau et les ingrédients habituels de telles lotions, une proportion assez élevée d'alcool.

Le document GB-A-2.056.854 décrit des cosmétiques, notamment des lotions et des shampoings, contenant, outre les ingrédients normaux inhérents à de tels produits, des proportions très élevées de lait (40 - 80%) et, comme agent de stabilisation, du propylène glycol.

Le document GB-A-2.051.076 décrit un procédé de préparation d'agents humidifiants utiles dans le domaine des cosmétiques, ces agents étant dérivés du lait écrémé par fermentation.

Plus précisément, ce document décrit la formation d'hydrolysats hydrosolubles de caséines par l'action de ferments lactiques et de protéases. Ces hydrolysats, incolores et transparents, sont ensuite incorporés, au taux de 0,1 à 10% en poids à des cosmétiques, notamment des lotions adoucissantes pour la peau, des crèmes, des produits de savonnerie, etc... de manière à conférer, à ces cosmétiques, des propriétés améliorées en ce qui concerne le pouvoir adoucissant, la prévention de dessèchement de la peau et le maintien de son élasticité.

Le document GB-A-2.037.160 décrit un produit lyophilisé dérivé du lait présentant des propriétés cosmétiques avantageuses, notamment pour l'humidification et l'assouplissement de la peau ainsi que sa régénération. Un tel produit contient principalement du lactose, des protéines du lait et de l'eau. On l'obtient par fermentation de lait écrémé ou non au moyen d'enzymes et de ferments lactiques, notamment la pancréatine, la pepsine, les ferments Lactobacillus Bulgaricus et Streptococcus Thermopilus. Le produit fermenté est ensuite lyophilisé et utilisé, en mélange, avec des compositions cosmétiques pour en améliorer les propriétés.

Le document DE-A-2.623.250 décrit des compositions cosmétiques contenant du lait. Parmi celles-ci, on cite les shampoings, les lotions de beauté, les crèmes à raser, les crèmes pour la peau, etc... Tous ces produits ont en commun, outre le lait et de l'eau, des produits tensio-actifs tels que les alcoyl-sulfonates, des acides gras sulfonés, des sels d'amines tertiaires, etc. Le lait peut se présenter sous forme de lait entier, lait maigre, lait acidifié, lait écrémé, yoghurt, crème, etc. sous forme aqueuse ou en poudre. Par ailleurs, les compositions décrites peuvent encore contenir, suivant l'usage auquel elles sont destinées, d'autres ingrédients tels que lanoline, parfums, glycérine et autres produits similaires.

Le document DE-A-2.650.560 décrit des compositions cosmétiques contenant du lait sous forme de yoghurt, celui-ci pouvant être utilisé à l'état frais ou en poudre. Parmi de tels cosmétiques, le document cite les produits liquides et solides, notamment les laits de beauté, les crèmes, les aérosols pour mousses. Ces compositions contiennent, outre le yoghurt, des agents conservateurs, des antioxydants, des chélatants et des inhibiteurs de protéases.

Le document EP-A-46.326 divulgue des compositions cosmétiques applicables sur la peau ou les cheveux contenant de la caséine sous forme de lait ou d'autres protéines du lait et, plus particulièrement, du petit-lait. Ces ingrédients dérivés du lait comprennent le petit-lait, le petit-lait délactosé, le petit-lait déminéralisé et leurs concentrats préparés par des procédés chimiques ou physiques, de même que le précipité résultant de la neutralisation du petit-lait, le petit-lait enrichi de calcium et les sous-produits du petit-lait résultant de la séparation

2

des produits susmentionnés.

Parmi les cosmétiques susceptibles d'être améliorés par les ingrédients dérivés du petit-lait, ce document cite les produits pour l'entretien de la peau et des cheveux, notamment shampoings, fixatifs, lotions capillaires, produits conditionneurs, produits pour permanentes, teintures, décolorants, lotions corporelles, cold cream, crèmes nettoyantes, émollients, détergents, savons, produits de maquillage, rouges à lèvres, produits de rasage, antisolaires, écrans, dépilatoires, etc...

En général, pour la formulation de tels cosmétiques modifiés, on ajoute, au mélange des ingrédients de ceux-ci, les dérivés du petit-lait susmentionnés, soit sous forme de solutions aqueuses, soit sous forme solide. Pour assurer la conservation de tels cosmétiques, le document recommande l'addition d'agents inhibiteurs ou germicides.

Bien que les cosmétiques décrits dans l'art antérieur décrit ci-dessus présentent, après modification par apport du lait ou de dérivés du lait, d'intéressantes propriétés nouvelles et des améliorations indéniables, ils ne sont cependant pas dépourvus de certains inconvénients parmi lesquels on peut citer:

1) Leur composition est souvent compliquée et onéreuse à réaliser, non pas en tant qu'ingrédients cosmétiques habituels, mais bien de par la nature des additifs dérivés du lait eux-mêmes, par exemple prétraitements subis par ceux-ci ou mode d'extraction à partir du lait naturel.

2) La durée de stockage à température ambiante est souvent limitée et le taux d'agents conservateurs (par exemple, alcool) nécessaire à leur conservation est souvent élevé ce qui peut compromettre, tout au moins en partie, les avantages d'adoucissement de la peau qui découlent de la présence des composés dérivés du lait.

3) L'aspect général du produit est quelquefois peu engageant, notamment en ce qui concerne un manque de réflexion de la lumière par les constituants de la composition, défaut qu'il faut alors corriger par incorporation d'un pigment, notamment le $TiO_2$. Il est en effet évident qu'un produit contenant des produits issus du lait se doit de rappeler, au premier coup d'oeil par son aspect et sa couleur, qu'il contient, en effet, de tels produits.

4) Les changements de couleur. Bien souvent les produits cosmétiques contenant du lait ont tendance à jaunir avec le temps et, pour cela, deviennent invendables en dépit de leurs qualités intrinsèques inchangées.

Pour ces diverses raisons, des recherches ont été entreprises pour mettre à jour des compositions nouvelles de produits cosmétiques contenant des dérivés du lait, ces compositions ayant réellement l'aspect de lait ou de mélanges contenant du lait, se conservant bien au stockage, relativement simples à formuler et présentant, bien entendu, tous les avantages reconnus des cosmétiques réalisés à base de lait.

Ces recherches ont abouti aux produits dont la définition est conforme à la revendication 1 annexée.

On a en effet constaté avec étonnement qu'à partir de solutions limpides de caséinate de soude contenant au moins un agent tensio-actif ou surfactant, on pouvait obtenir des solutions laiteuses stables rappelant à s'y méprendre l'aspect du lait naturel, par addition de calcium, notamment de sels solubles comme $CaCl_2$ dans l'intervalle de pH indiqué. Une telle opération permet en fait la reformation des micelles de protéines du lait, celles-ci se trouvant sous la forme de phosphocaséinate de calcium en suspension micellaire et colloïdale. Il est intéressant de noter à ce propos qu'en l'absence d'un surfactant, une telle adjonction conduit plutôt à la précipitation de phosphocaséinate calcique.

L'utilisation de caséine industrielle, soit pure soit conjointement avec les autres protéines lactées (albumines, globulines, protéines du sérum), présente les avantages d'un produit de nature essentiellement constante, stable, relativement peu onéreux et pratiquement exempt de lactose. C'est en effet principalement au lactose que sont dûs les effets de jaunissement au stockage et d'altération qu'on observe dans le cas de cosmétiques contenant du lait ou de produits du lait. Il est donc possible, grâce à l'emploi de caséine dont les propriétés et la nature restent invariables (dans certaines limites naturellement), de fournir des cosmétiques contenant des produits dérivés du lait dont la composition et les performances sont stables d'une fabrication à l'autre, ce qui est extrêmement difficile à garantir en partant de lait naturel.

On peut utiliser la solution colloïdale de phosphocaséinate de calcium dont il est question ici pour modifier avantageusement la plupart des produits cosmétiques modifiables par apport de lait ou d'ingrédients dérivés du lait et cités dans l'art antérieur. On a donné notamment une liste non-exhaustive de tels produits avec référence au document EP-A-46.326. Cependant, les cosmétiques qu'on préfère suivant cette invention sont les produits capillaires ou à usage corporel comme les savons, shampoings, lotions, crèmes, produits de bain, etc... De manière générale, on peut incorporer à de tels cosmétiques des quantités de protéines lactées (sous la forme préconisée) pouvant atteindre 20% en poids sans inconvénient majeur relatif à la couleur, la consistance ou la stabilité au stockage. Grâce à l'emploi de caséine purifiée, l'adjonction de germicides et autres biocides peut rester minimale.

Parmi les produits de l'invention qu'on préfère, on mentionne les produits pour l'entretien de la peau et des cheveux, notamment les produits pour la douche, le bain, les shampoings, etc... De tels produits contiennent généralement des agents tensio-actifs (surfactants anioniques, cationiques et non-ioniques, produits de détergence), des émollients et facteurs hydratants, (matières grasses, acides gras, constituants protéiques), des biocides (germicides, bactéricides, fongicides, etc...) des parfums, des azurants et opalescents optiques, des opacifiants, etc...

Suivant une formule préférée d'un shampoing, par exemple, celui-ci contient, en poids, les ingrédients suivants: agents tensio-actifs anioniques 10 - 50%, acides gras naturels 0,5 - 5%, protéines dérivées du lait 3 - 15%, ion calcium 0,1 - 1%, le reste à 100% étant constitué par l'eau de la solution et au moins un ingrédient additionnel choisi parmi les biocides, les parfums, les azurants, les opacifiants, les agents opalescents et iridisants et les agents tampons. De préférence le pH d'un tel mélange est de 6,5 à 7 et sa viscosité est de 3 à 6 Pa.s (3000 à 6000 cP).

Parmi les agents tensio-actifs convenant à une telle formulation, on cite les sodio- et ammonio-sulfates et sulfonates d'alcools gras et de polyoxyalcoylènes, par exemple sodio-sulfate de lauryle ou de polyoxyéthylène glycol. Cependant d'autres surfactants peuvent aussi convenir, par exemple les surfactants non-ioniques.

Parmi les acides gras naturels, on cite les acides oléique, ricinoléique, myristique, linoléique et stéarique.

Parmi les biocides, on cite les sels d'amines quaternaires, les esters p-hydroxybenzoïque, l'imidazolidinylurée, le phénoxyéthanol, les dérivés d'isothiazolinone, le formaldéhyde et les acides benzoïque, salicylique et sorbique.

Pour préparer les cosmétiques suivant la présente invention on aura avantageusement recours au procédé défini à la revendication 6. On notera que le terme caséine ou caséine soluble désigne, au choix, la caséine sodique, potassique ou d'ammonium, ou la caséine acide dissoute en milieu alcalin.

Ainsi, par exemple dans le cas de la préparation d'une composition de shampoing telle que définie à la revendication 4, ou pourra opérer de la manière définie à la revendication 7. De préférence on utilise, comme sel de calcium, le $CaCl_2$ par exemple, sous forme de $CaCl_2.2H_2O$; cependant d'autres sels de calcium hydrosolubles peuvent aussi être employés.

Les exemples qui suivent illustrent l'invention. Les marques commerciales déposées sont reconnues comme telles.

Exemple 1 : Préparation d'une composition de shampoing.

Dans 740 g d'eau, on a dispersé à froid 72,1 g de caséinate de soude (AME-100, fourni par l'Union Centrale des Producteurs Suisses de lait) puis on a chauffé ce mélange à 70°C dans un homogénéiseur jusqu'à dissolution complète. On a ensuite ajouté à la solution 120 g de sulfate de sodium et de lauryle (Texapon-Z, fourni par Impag A.G., Zurich) et 24 g d'acide oléique.

On a alors abaissé la température à 50°C et on a ajouté 3 g d'imidazolidinyl-urée et 5 g de p-hydroxybenzoate de phénoxyéthyle; puis on a ajusté le pH à 6,75 au moyen d'une solution aqueuse de KOH à 20% en poids et on a ajouté, goutte à goutte sous vigoureuse agitation et refroidissement, 20,5 g d'une solution aqueuse à 64,8% en poids de $CaCl_2.2H_2O$

Le mélange, de limpide, s'est transformé en une masse laiteuse de couleur blanche intense qu'on a ensuite parfumée par adjonction de 4 g d'un parfum (Freezia, fourni par GIVAUDAN S.A., Genève). Cette composition de shampoing a été testée suivant les moyens habituels utilisés en cosmétique et a démontré un excellent pouvoir de lavage tout en préservant la chevelure et en lui donnant un éclat exceptionnel.

Des essais de stockage de ce shampoing à 40°C n'ont montré aucune tendance au floconnage ou séparation de phases. Tout au plus a-t-on-observé un léger affaiblissement de réflexion de la lumière, effet qui a d'ailleurs disparu au refroidissement.

Exemple 2: Préparation d'une composition pour bains.

On a mélangé de façon intime à 70°C dans un homogénéiseur les ingrédients suivants:

| Ingrédients | g |
|---|---|
| Eau | 717 |
| Caséinate de sodium (AME-100) | 162 |
| Texapon-Z | 78 |
| Acide oléique; | 38 |
| on a ajouté ensuite à 50°C: | |
| Phenonip (Nipa, GB) | 5 |

Ce mélange a été dénommé prémélange A. On a ensuite constitué, à 70°C, un produit pour le bain par adjonction successive des ingrédients suivants (% en poids)

| Ingrédients | % |
|---|---|
| Eau | 6,32 |
| SAG-10 (antimousse silicone de Union-Carbide) | 0.15 |
| Texapon-Z (Henkel) | 8,87 |
| Texapon-N (Henkel) | 37,51 |
| Neo-Purcellin-SE (O/W-2/066280) (émulsificateur fourni par DRAGOCO, Allemagne) | 2,0 |
| Prémélange A | 35,0 |
| Euperlan PK-771 (agent moirant fourni par HENKEL, Allemagne) | 3,0 |
| KOH (solution à 7,4%); | 0,62 |
| puis, après avoir refroidi à environ 50°C, on a encore ajouté: | |
| Phenonip-100 | 0,2 |
| Biopur-100 | 0,2 |
| $CaCl_2.2H_2O$ (solution à 64,8%) | 3,63 |
| Parfum | 2,5 |

On a ensuite homogénéisé le tout 5 min à 7000 t/min ce qui a fourni une composition de pH 6,75.

En utilisant dans l'eau le produit ci-dessus, dont l'aspect est d'un blanc laiteux soutenu avec des reflets irisés, on a obtenu un bain moussant et adoucissant très agréable.

Exemple 3 : Préparation d'une crème pour la peau

Dans un mélangeur, on a dissous 45,6 g de caséine dans 548,8 g d'eau, puis on a porté la solution à 70°C et on y a ajouté, en agitant, 24 g de glycérol. Cette solution a été identifiée comme M-1.

Dans un mélangeur (type Sovirel SVS-295), on a mélangé à 70°C 32 g de Cutina KD-16 et 24 g de Lanette 0. Puis, après 30 min d'agitation, on a ajouté 80 g de Cétiol V et 8 g d'huile d'amandes douces. On a agité 15 min à 70°C et on a encore ajouté 5,6 g de lanoline anhydre et 8 g d'huile de ricin. Ce mélange a été identifié comme M-2.

Tout en agitant doucement à 70°C, on a incorporé lentement le produit M-2 au produit M-1. On a alors abaissé la température à 50°C et ajouté, successivement, tout en agitant, 16 g d'Hydroviton, 1 g d'acide lactique, 2 ml de solution de chlorure de calcium à 64,8 %, 0,8 g de Kathon CG et 4 g de parfum. On a alors abaissé la température à 40°C et procédé à une homogénéisation 5 min à 7000 rpm.

On a ainsi obtenu une crème revitalisante fluide permettant d'adoucir la peau après le bain et de la rendre souple et douce, tout en donnant une agréable impression de fraîcheur. Cette crème convient aux peaux même les plus sensibles.

## Revendications

1. Produit cosmétique et/ou de savonnerie dont l'aspect rappelle celui du lait naturel et contenant, outre les ingrédients habituels d'un tel produit, au moins une substance dérivée du lait, caractérisé en ce que cette substance est constituée de caséine ou d'un mélange de caséine et d'autres protéines lactées, cette caséine étant présente, au moins en partie, sous forme de solution colloïdale et micellaire de phosphocaseinate de calcium à un pH de 6 à 7.

2. Produit cosmétique suivant la revendication 1, caractérisé en ce qu'il est exempt de lactose ou, tout au moins, que la quantité de celui-ci en poids de la composition ne dépasse pas 0,1%.

3. Produit cosmétique suivant la revendication 1, caractérisé en ce que son contenu en protéines d'origine lactée est de 3 à 20% en poids.

4. Produit cosmétique suivant la revendication 1, en ce qu'il consiste en un shampoing contenant, en poids: agent tensio-actif anionique 10 - 50%, acide oléique 0,5 - 4%, protéines dérivées du lait 3 - 15%, ion $Ca^{++}$ 0,1 - 1%, le reste à 100% étant constitué par de l'eau et un ou plusieurs ingrédients additionnels choisis parmi les biocides, les parfums, les azurants, les opacifiants et les agents tampons.

5. Produit cosmétique suivant la revendication 4, caractérisé en ce que sa viscosité à température ambiante est de 3 à 6 Pa.s (3000 à 6000 cP).

6. Procédé pour préparer un produit cosmétique suivant la revendication 1, caractérisé en ce qu'on incorpore de façon homogène à au moins une partie des ingrédients constitutifs habituels dudit produit, ceux-ci comprenant au moins un tensio-actif, une solution aqueuse limpide de caséine ou caseinate soluble contenant, ou non, d'autres protéines d'origine lactée, puis on ajoute une quantité d'ions calcium suffisante pour qu'à pH 6 - 7 le caséinate soluble se convertisse en un colloïde micellaire de phosphocaseinate calcique, puis, le cas échéant, on ajoute les ingrédients constitutifs restants du cosmétique.

7. Procédé suivant la revendication 6, appliqué à la préparation d'un shampoing suivant la revendication 4, carac en ce qu'on mélange à une température de 50 - 80°C tout ou partie de l'agent tensio-actif et de l'acide oléique à une solution aqueuse de caséinate de soude, on ajoute la plupart ou tous les autres ingrédients à l'exception des ions $Ca^{++}$, puis, entre 40 et 60°C et à un pH ajuste à une valeur entre 6 et 7, on ajoute lentement une solution aqueuse concentrée d'un sel de calcium hydrosoluble ce qui convertit la solution limpide en une solution laiteuse à haut pouvoir photoréflectif.

8. Procédé suivant la revendication 7, caractérisé en ce que le sel de calcium est le chlorure de calcium.

## Patentansprüche

1. Kosmetik- und/oder Seifenprodukt, dessen Aussehen an das von natürlicher Milch erinnert und das, außer den herkömmlichen Bestandteilen eines solchen Produkts, zumindest eine aus Milch stammende Substanz enthält, dadurch gekennzeichnet, daß diese Substanz aus Kasein oder aus einer Mischung von Kasein mit anderen Milchproteinen gebildet wird, wobei diese Kasein, zumindest teilweise, in Form einer kolloidalen und micellaren Lösung von Kalziumphosphokaseinaten mit einem pH-Wert von 6 bis 7 vorhanden ist.

2. Kosmetikprodukt gemäß Anspruch 1, dadurch gekennzeichnet, daß es frei von Laktose ist oder, daß wenigstens ihre Menge im Gewicht der Zusammensetzung nicht 0,1% übersteigt.

3. Kosmetikprodukt gemäß Anspruch 1, dadurch gekennzeichnet, daß sein Gehalt an Proteinen aus Milch 3 bis 20 Gew.-% ist.

4. Kosmetikprodukt gemäß Anspruch 1, dadurch gekennzeichnet, daß es aus einem Shampoo besteht, welches, in Gew.-%, enthält: 10 - 50% anionisches, grenzflächenaktives Mittel, 0,5 - 4% Oleinsäure, 3 - 15% aus Milch stammende Proteine, 0,1 - 1% $Ca^{++}$-ionen, der Rest auf 100% wird aus Wasser und ein oder mehreren zusätzlichen Bestandteilen, die aus den Bioziden, den Duftstoffe, den Aufhellern, den Trübungsstoffen und den Pufferstoffen ausgewählt werden, gebildet.

5. Kosmetikprodukt gemäß Anspruch 4, dadurch gekennzeichnet, daß seine Viskosität bei Raumtemperatur 3 bis 6 Pa.s (3000 bis 6000 cP) ist.

6. Verfahren zur Herstellung eines Kosmetikprodukts gemäß Anspruch 1, dadurch gekennzeichnet, daß man in homogener Weise in zumindest einen Teil der herkömmlichen, wesentlichen Bestandteile des genannten Produkts, welche zumindest ein grenzflächenaktives Mittel enthalten, eine klare, wässrige Lösung von Kasein oder löslichem Kaseinat einbringt, das andere Proteine aus Milch enthält oder nicht, daß man dann eine genügende Menge Kalziumionen hinzufügt, sodaß bei einem pH-Wert von 6 bis 7 das lössliche Kaseinat in ein micellares Kalziumphosphokaseinat-Kolloid übergeht, und daß man dann, gegebenenfalls, die übrigen, wesentlichen Kosmetikbestandteile zugibt.

7. Verfahren gemäß Anspruch 6 angewandt auf die Herstellung eines Shampoos gemäß Anspruch 4, dadurch gekennzeichnet, daß man bei einer Temperatur von 50 - 80°C alles oder Teil des grenzflächenaktiven Mittels und der Oleinsäure in eine wässrige Lösung von Kaseinatsoda mischt, daß man einen Großteil von den alle anderen Bestandteile mit Ausnahme der Ca$^{++}$-ionen hinzufügt, dann, zwischen 40 und 60°C und bei einem pH-Wert, der auf einen Wert zwischen 6 und 7 eingestellt wurde, langsam eine wässrige, konzentrierte Lösung eines wasserlöslichen Kalziumsalzes hinzugibt, was die klare Lösung in eine milchige Lösung mit großem Photoreflexionsvermögen umwandelt.

8. Verfahren gemäß Anspruch 7, dadurch gekennzeichnet, daß das Kalziumsalz Kalziumchlorid ist.

## Claims

1. A cosmetic and/or a soap product whose appearance brings to mind that of natural milk and containing, other than the usual ingredients of such a product, at least one substance derived from milk, characterised in that this substance is formed from casein or from a mixture of casein and other milk proteins, this casein being present, at least in part, in the form of a colloidal and micellar solution of calcium phosphocaseinate at a pH of from 6 to 7.

2. A cosmetic product according to Claim 1, characterised in that it is free from lactose, or, at the very least, that the quantity by weight of lactose in the composition does not exceed 0.1%.

3. A cosmetic product according to Claim 1, characterised in that its content of proteins of milk origin is from 3 to 20% by weight.

4. A cosmetic product according to Claim 1, characterised in that it consists of a shampoo containing, by weight: anionic surface-active agent 10-50%, oleic acid 0.5-4%, proteins derived from milk 3-15%, Ca$^{++}$ ion 0.1 - 1%, the remainder to 100% being formed of water and one or several additional ingredients selected from among biocides, perfumes, blueing agents, opacifiers, and buffering agents.

5. A cosmetic product according to Claim 4, characterised in that its viscosity at ambient temperature is from 3 to 6 Pa·s.

6. A process for preparing a cosmetic product according to Claim 1, characterised in that at least a part of the usual constituent ingredients of the said product are incorporated in a homogeneous manner, these including at least a surface-active agent, a clear aqueous solution of casein or soluble caseinate optionally containing other proteins of milk origin, then a sufficient quantity of calcium ions is added so that at pH 6-7 the soluble caseinate is converted into a micellar colloid of calcium phosphocaseinate, then, if necessary, the remainder of the constituent ingredients of the cosmetic are added.

7. A process according to Claim 6, applied to the preparation of a shampoo according to Claim 4, characterised in that all or part of the surface-active agent and of the oleic acid are mixed at a temperature of 50-80°C with an aqueous solution of sodium caseinate, the greater part or all of the other ingredients with the exception of the Ca$^{++}$ ions are added, then, between 40° and 60°C and at a pH adjusted to a value between 6 and 7, a concentrated aqueous solution of a water soluble calcium salt is slowly added, which converts the clear solution into a milky solution of high light reflective power.

8. A process according to Claim 7, characterised in that the calcium salt is calcium chloride.